# EUROPÄISCHE PATENTSCHRIFT

(11) **EP 2 020 580 B1**
(45) Veröffentlichungstag und Bekanntmachung des Hinweises auf die Patenterteilung: **02.09.2015**
(21) Anmeldenummer: 08013152.7
(22) Anmeldetag: 22.07.2008
(51) Int. Cl.: F26B 5/14, F26B 7/00, F26B 23/00

(54) **Verfahren zur Aufbereitung von Silage**
Method for processing silage
Procédé de préparation d'ensilage

(30) Priorität: 03.08.2007 DE 102007036729
(43) Veröffentlichungstag der Anmeldung: 04.02.2009
(73) Patentinhaber: GETproject GmbH & Co. KG, 24109 Kiel (DE)
(72) Erfinder: Götz, Johann, 24105 Kiel (DE)
(74) Vertreter: Lobemeier, Martin Landolf

(56) Entgegenhaltungen:
- EP-A1- 1 211 308
- DE-A1- 19 615 551
- DE-C1- 19 809 400
- US-A- 5 135 861
- US-A1- 2006 093 713

## Beschreibung

Die Erfindung betrifft ein Verfahren zur Trocknung von Biomasse. Desweiteren betrifft die Erfindung eine Anlage zur Aufbereitung von Biomasse für die Energieerzeugung mit einem Maischbehälter, einer mit dem Maischbehälter verbundenen Entwässerungsvorrichtung und einem mit der Entwässerungsvorrichtung verbundenen Trockner.

Aus der EP 1 829 829 A2 ist ein Verfahren zur Erzeugung von Biogas aus silierter Biomasse bekannt, bei dem die Biomasse nach einer Konditionierung in einem Mischbehälter, in dem der Aufschluss der Biomasse mit erhitzter Prozessflüssigkeit erfolgt, durch mechanische Entwässerung mit Hilfe von Pressen in einen Feststoff, der zu einem großen Teil aus der Zellstruktur der als Biomasse verwendeten Pflanzen besteht und in eine flüssige Phase mit gelösten und ungelösten organischen und anorganischen Pflanzeninhaltsstoffen getrennt wird. Während der Feststoff in einem Trockner getrocknet und zu Brennstoffe (z.B. Pellets) verarbeitet werden kann, die einer Biomassefeuerungsanlage zugeführt werden können, wird lediglich die flüssige Phase in den Fermenter einer Biogasanlage zur Erzeugung von Biogas eingebracht.

Im Vergleich zu einer üblichen Ganzpflanzenvergärung hat die Auftrennung in eine feste und in eine flüssige Phase den Vorteil, dass die Vergärung der flüssigen Phase mit sehr kurzen Verweilzeiten erfolgen kann. Weiterhin werden auch keine Rührwerke benötigt, sondern es muss lediglich für eine geeignete Umwälzung der Fermenterinhalte gesorgt werden, damit Sinkstoffe wieder in Suspension gebracht werden können. Die überschüssige Abwärme der üblicherweise zur Erzeugung von Strom aus dem durch die Vergärung gewonnenen Biogas verwendeten Blockheizkrafrwerke wird vollständig zur Trocknung des Feststoffes genutzt, was bei einer konventionellen Ganzpflanzenvergärung auf Grund örtlicher Gegebenheiten kaum möglich ist. Mit dem aus dem Feststoff hergestellten Brennstoff steht eine lager- und transportfahiger Energieträger zur Verfügung.

Nachteilig an diesem Vorgehen ist, dass bei der Trocknung der Feststoffe stark geruchsbehaftete Bestandteile der silierten Pflanzen bei den üblichen Trocknungstemperaturen von 50 bis 150 °C vermehrt in Form von Dampf in die Luft übergehen. Daher sind regelmäßig aufwändige Filtersysteme notwendig, die die unmittelbare Geruchsbelastung in der Umgebung der Anlagen nach dem eingangs genannten Verfahren auf ein vertretbares Maß reduzieren. Mit den dampfförmigen Pflanzeninhaltsstoffen gehen auch energiehaltige Anteile der Pflanzen verloren.

Aufgabe der Erfindung ist es daher, ein Verfahren zu entwickeln, das die Geruchsbelästigung bei der Trocknung von Biomasse zur Produktion von festem Brennstoff reduziert. Desweiteren soll auch der größte Teil des in der Trocknerabluft enthaltenen Wassers kondensiert werden, so dass er nicht in Form von Dampf oder Nebel an die Atmosphäre abgegeben wird.

Die Erfindung wird durch das Verfahren mit den Merkmalen des Anspruch 1 gelöst. Die Unteransprüche geben vorteilhafte Ausgestaltungen des Verfahrens an.

Grundgedanke der Erfindung ist es, die beim Trocknen des Feststoffes anfallende warme und feuchte Trocknerabluft mit der aufzubereitenden kalten Biomasse in Kontakt zu bringen, wodurch das in der Trocknerabluft enthaltenen Wasser und insbesondere die zur Geruchsbelästigung in der Umgebung der Trocknung beitragenden Substanzen im Wesentlichen an der kalten Biomasse kondensieren und somit nicht in die Umgebung gelangen.

Ein weiterer, damit verbundener positiver Effekt ist es, dass die warme Trocknerabluft die frisch zugeführte Biomasse erwärmt und dadurch die für das Maischen der Biomassen sowie die in der Trocknungsphase zum Erwärmen des Feststoffes aufzubringende Energie verringert ist, da die Biomassen und der Feststoff bereits beim Eintritt in den Maischbehälter bzw. in den Trockner höhere Temperaturen aufweisen als ohne Abkühlung der Trocknerabluft und Kondensation der dampfförmigen Inhaltsstoffe an der kalten Biomasse.

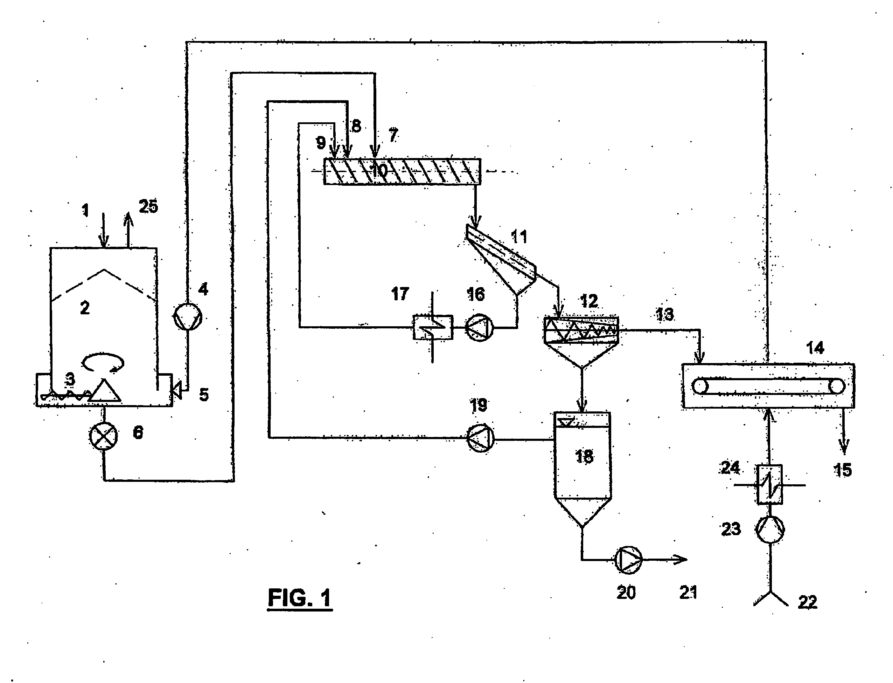

Die Erfindung wird im Folgenden anhand der einzigen Fig. 1 näher erläutert:
Fig. 1 zeigt eine schematische Übersicht über den Stofffluss in einer Anlage zur Aufbereitung von Biomasse für die Energieerzeugung unter Verwendung von Komponenten der Anlage nach der Erfindung.

Biomasse, bevorzugt silierte Energiepflanzen (im Folgenden: Silage), wird über eine Silageeintrittsöffnung 1 in einen Kondensationsbehälter 2 gebracht und mit einer Austragschnecke 3 über die gesamte Bodenfläche des Kondensationsbehälters 2 gleichmäßig ausgetragen. Über eine Austragsschleuse 6 kann dem Kondensationsbehälter 2 Silage entnommen werden, wobei durch eine geeignete Steuerung von Materialzulauf und Materialabfluss ein bevorzugt konstanter Füllstand im Kondensationsbehälter sichergestellt werden kann.

Die dem Kondensationsbehälter 2 entnommene Silage wird einem Maischbehälter 10 über eine den Kondensationsbehälter 2 mit dem Maischbehälter 10 verbindende Leitung zugeführt und mit Wasser versetzt. Die Maische wird nach einer vorbestimmten Verweilzeit im Maischbehälter 10 zur Abscheidung von freiem Wasser in einen Wasserabscheider 11 gefördert. In der Schneckenpresse 12 wird anschließend das an dem Maischgut anhaftende Oberflächenwasser abgepresst und der überwiegende Teil des in der Pflanzenstruktur eingeschlossenen Wassers zusammen mit organischen und anorganischen Pflanzeninhaltsstoffe ausgepresst (im Folgenden Presssaft genannt).

Das in dem Wasserabscheider 11 abgeschiedene Wasser wird über die Maischwasserrückführung 9 wieder dem Maischebehälter 10 zugeführt. Durch eine Wasseraufnahme der Biomasse durch das Maischen und das an den Oberflächen des Maischgutes anhaftende Wasser entsteht in dem Maischbehälter ein Wassermangel, der durch Frischwasser ausgeglichen werden müsste. Ersatzweise wird dieser Wassermangel wird mit einer um nicht gelöste Pflanzeninhaltsstoffe abgereicherten Fraktion des Presssaftes aus dem Presssaftbehälter 18 ausgeglichen. Zu diesem Zweck wird der Presssaftbehälter 18 als Sedimentationseinrichtung ausgeführt. Damit sind besonders bevorzugt zwei Wasserkreisläufe hergestellt, die den Wasserverbrauch des erfindungsgemäßen Verfahrens zu minimieren helfen.

Weiterhin kann der Presssaftbehälter 18 als Zwischenspeicher ausgeführt werden, der den in der Entwässerungsschnecke 12 abgeschiedenen Presssaft, in dem die organischen und anorganischen Pflanzenbestandteile enthalten sind, aufnimmt. Dieser Presssaft kann dosiert entnommen und mittels einer Förderpumpe 20 dem Fermenter 21 einer Biogasanlage (nicht gezeigt) zur Erzeugung von Biogas zugeleitet werden, wobei die erfindungsgemäße Anlage selbst als Teil einer Biogasanlage verstanden werden, also Teil einer Biogasanlage sein kann.

Die in der Entwässerungsstufe 11, 12 anfallende entwässerte Maische (im Folgenden: Feststoff), wird über den Pressgutaustritt einem Trockner 14 zugeführt und getrocknet, wobei in herkömmlicher Art und Weise Luft aus der Umgebung über einen Trocknerlufteintritt 22 mittels eines Trocknerluftventilators 23 angesogen, in einer Trocknerlufterhitzung 24 auf wahlweise ca. 50 bis 150 °C erwärmt und dem Trockner 14 zugeführt wird. Der im Trockner 14 getrocknete Feststoff kann dem Trockner 14 nach einer vorbestimmten Zeit mit einem vorbestimmten Trocknungsgrad entnommen werden und beispielsweise als Brennstoff verwendet werden.

Im Gegensatz zu herkömmlichen Anlage und Verfahren ist nun nach dem Verfahren und der Anlage gemäß der Erfindung vorgesehen, dass die im Trockner 14 anfallende Trocknerabluft dem Kondensationsbehälter 2 zugeführt wird, beispielsweise mithilfe eines Ventilators 4. Dabei wird die Trocknerabluft vom Trockner 14 in den Kondensationsbehälter 2 über einen am Kondensationsbehälter 2 in Bodennähe des Kondensationsbehälters 2 bevorzugt als Ringkanal ausgebildeten Lufteintritt 5 eingeblasen. Dadurch wird sichergestellt, dass die Trocknerabluft die in den Kondensationsbehälter 2 eingefüllte Silage im gesamten Umfang des Kondensationsbehälters 2 im Gegenstrom durchströmen muss, d.h. der Silagefluss wird im Kondensationsbehälter 2 im Wesentlichen von oben nach untern verlaufen, während die Trocknerabluft den Kondensationsbehälter 2 im Wesentlichen von unten nach oben durchströmen wird. Dabei kondensieren in der Trocknerabluft enthaltener Wasserdampf und die darin enthaltene dampfförmige Pflanzeninhaltsstoff an der kalten Silage, wobei die Trocknerabluft gleichzeitig die Silage erwärmt. Die ansonsten über die Trocknerabluft entschwindende energetisch nutzbaren und geruchsbehafteten Pflanzeninhaltsstoffe werden zurück gewonnen und der Energieproduktion wieder zugeführt.

Erfindungsgemäß kann damit die Geruchsbelästigung der Umwelt durch Trocknungsvorgänge von Biomasse, insbesondere von entwässerter Silage, reduziert werden, da die geruchsauslösenden Stoffe im Wesentlichen im Stoffkreislauf der erfindungsgemäßen Anlage fixiert werden. Sollten doch in Teilbereichen der Anlage Filter notwendig sein, so wird jedenfalls die Belastung der Filter durch geruchsauslösende Stoffe der Trocknerabluft verringert, wodurch unmittelbar Kosten bei der Dimensionierung derartiger Filteranlagen und/oder deren Wartung eingespart werden können.

### Bezugszeichenliste:

- 1: Silageeintrittsöffnung
- 2: Kondensationsbehälter
- 3: Austragschnecke
- 4: Ventilator
- 5: Lufteintritt
- 6: Austragsschleuse
- 7: Eintritt vorgewärmter Silage
- 8: Presswasserrückführung
- 9: Maischwasserrückführung
- 10: Maischbehälter
- 11: Wasserabscheider
- 12: Entwässerungspresse
- 13: Pressgutaustritt
- 14: Trockner
- 15: Trockengutaustritt
- 16: Prozesswasserpumpe
- 17: Wärmetauscher
- 18: Presssaftbehälter
- 19: Presssaftrückführungspumpe
- 20: Presssaftförderpumpe zur Fermentation
- 21: zum Fermenter der Biogasanlage
- 22: Trocknerlufteintritt
- 23: Trocknerluftventilator
- 24: Trocknerlufterhitzung
- 25: Trocknerluftaustritt

## Patentansprüche

1. Verfahren zur Trocknung von Silage, **gekennzeichnet durch** die Schritte:
- Einfuhren von Silage in einen Kondensationsbehälter (2),
- Fördern der Silage aus dem Kondensationsbehälter (2) in einen Maischbehälter (10),
- Fördern der Maische aus dem Maischbehälter (10) in eine Entwässerungsvorrichtung (11, 12),
- Entwässern der Maische in der Entwässerungsvorrichtung (11, 12),
- Fördern der entwässerten Maische in einen Trockner (14) und
- Trocknen der entwässerten Maische,
wobei die Trocknerabluft zur Reduzierung der Geruchsbelastung der Umwelt durch Kondensation von in der Abluft abgeführter Substanzen in den Kondensationsbehälter (2) mit frisch zugeführter Silage in Kontakt bringend geführt wird.

2. Verfahren nach Anspruch 1, **dadurch gekennzeichnet, dass** die Maische mittels eines Wasserabscheiders (11) und/oder einer Entwässerungspresse (12) entwässert wird.

3. Verfahren nach einem der vorhergehenden Ansprüche, **dadurch gekennzeichnet, dass** das Entwässern in der Entwässerungsvorrichtung (11, 12) unter Abscheiden und/oder Auspressen von Wasser, gelösten und ungelösten, organischen und anorganischen Pflanzenbestandteilen aus der Zellstruktur der Silage erfolgt.

4. Verfahren nach einem der vorhergehenden Ansprüche, **dadurch gekennzeichnet, dass** wenigstens ein Teil der in der Entwässerongsvorrichtung (11,12) abgeschiedenen flüssigen Phase in den Maischbehälter (10) zurückgeführt wird.

5. Verfahren nach Anspruch 4, **dadurch gekennzeichnet, dass** die in den Maischbehälter (10) zurück geführte flüssige Phase mit einem Wärmetauscher (17) beheizt wird.

6. Verfahren nach einem der vorhergehenden Ansprüche, **dadurch gekennzeichnet, dass** ein Presssaftbehälter (18) als Sedimentationstank ausgebildet wird.

7. Verfahren nach einem der vorhergehenden Ansprüche, **dadurch gekennzeichnet, dass** wenigstens ein Teil der flussigen Phase einem Fermenter einer Biogasanlage zugeführt wird.

## Claims

1. A method for drying silage, **characterized by** the following steps:
- introducing silage into a condensation container (2),
- conveying the silage from the condensation container (2) into a mash container (10),
- conveying the mash from the mash container (10) into a dewatering device (11, 12),
- dewatering the mash in the dewatering device (11, 12),
- conveying the dewatered mash into a dryer (14), and
- drying the dewatered mash,
the dryer exhaust air, for the purpose of reducing the emission of odours into the environment on account of the condensation of substances entrained in the exhaust air, being guided into the condensation container (2) while bringing it into contact with freshly fed silage.

2. The method according to Claim 1, **characterized in that** the mash is dewatered using a water separator (11) and/or a dewatering press (12).

3. The method according to one of the preceding claims, **characterized in that** dewatering in the dewatering device (11, 12) takes place by separating off and/or pressing out water, dissolved or undissolved organic and inorganic plant substances from the cell structure of the silage.

4. The method according to one of the preceding claims, **characterized in that** at least part of the liquid phase separated off in the dewatering device (11, 12) is fed back into the mash container (10).

5. The method according to Claim 4, **characterized in that** the liquid phase fed back into the mash container (10) is heated using a heat exchanger (17).

6. The method according to one of the preceding claims, **characterized in that** a press juice container (18) is formed as a sedimentation tank.

7. The method according to one of the preceding claims, **characterized in that** at least part of the liquid phase is fed to a fermenter in a biogas plant.

## Revendications

1. Procédé destiné au séchage d'ensilage, **caractérisé par** les étapes suivantes :
- Introduction de l'ensilage dans un réservoir de condensation (2),
- Transport de l'ensilage hors du réservoir de condensation (2) dans un réservoir à moût (10),
- Transport du moût hors du réservoir à moût (10) dans un dispositif de déshydratation (11, 12),
- Déshydratation du moût dans le dispositif de déshydratation (11, 12),
- Transport du moût déshydraté dans un séchoir (14) et
- Séchage du moût déshydraté,
l'air vicié du séchoir étant guidé en vue de la réduction de la nuisance olfactive à l'environnement par condensation des substances transportées conjointement dans l'air vicié dans le réservoir de condensation (2) en le mettant en contact avec de l'ensilage introduit fraîchement.

2. Procédé selon la revendication 1, **caractérisé en ce que** le moût est déshydraté au moyen d'un séparateur d'eau (11) et/ou d'une presse d'égouttage (12).

3. Procédé selon une quelconque des revendications précédentes, **caractérisé en ce que** la déshydratation est réalisée dans le dispositif de déshydratation (11, 12) en séparant et/ou en pressant de l'eau, des composants végétaux dissous et non dissous, organiques et anorganiques, issus de la structure cellulaire de l'ensilage.

4. Procédé selon une quelconque des revendications précédentes, **caractérisé en ce qu'**au moins une partie de la phase fluide séparée dans le dispositif de déshydratation (11, 12) est reconduite dans le réservoir à moût (10).

5. Procédé selon la revendication 1, **caractérisé en ce que** la phase fluide reconduite dans le réservoir à moût (10) est chauffée avec un échangeur thermique (17).

6. Procédé selon une quelconque des revendications précédentes, **caractérisé en ce qu'**un réservoir à jus de pression (18) est agencé comme citerne de sédimentation.

7. Procédé selon une quelconque des revendications précédentes, **caractérisé en ce qu'**au moins une partie de la phase fluide est introduite dans un fermenteur d'une installation de biogaz.
